# EUROPEAN PATENT SPECIFICATION

(11) **EP 0 590 084 B1**
(45) Date of publication and mention of the grant of the patent: **23.09.1998**
(21) Application number: 92914974.8
(22) Date of filing: 19.06.1992
(51) Int. Cl.: A61M 16/00, A61M 37/00

(54) **COMBINATION OF TRACHEOSTOMY TUBE AND OBTURATOR**
KOMBINATION EINES OBTURATORS UND EINER LUFTRÖHRENKANÜLE
COMBINATION D'OBTURATEUR ET DE TUBE DE TRACHEOSTOMIE

(30) Priority: 21.06.1991 US 717828
(43) Date of publication of application: 06.04.1994
(73) Proprietor: Smiths Industries Medical Systems, Inc., Keene, NH 03431-0724 (US)
(72) Inventor: CARR, Ian, R., Jaffrey, NH 03452 (US); LABOMBARD, Denis, Georgetown, MA 01833 (US)
(74) Representative: Coles, Graham Frederick
(86) International application number: US9205278
(87) International publication number: WO9300124

(56) References cited:
- GB-A- 2 224 213
- US-A- 3 794 041
- US-A- 4 231 365

## Description

This invention relates to combinations of a tracheostomy tube including a cannula and an obturator insertable into said cannula for guiding intubation of the tracheostomy tube, of the kind in which the cannula of the tracheostomy tube is curved with a non-constant radius and the obturator comprises an elongate, single-piece member of resilient and flexible material that is bendable to follow the curve on its insertion in the cannula, the elongate member involving shaft means which extends between proximal and distal ends of the member and which over a substantial part of its length is dimensioned to impart a degree of rigidity both in the plane of the curve and normal to it, and in which the shaft means includes a flexible portion which is of substantially rectangular cross-section with a width measured normal to said plane greater than its thickness, and which is located adjacent the distal end for easy flexing within said plane at that location.

Combinations of tracheostomy tube and obturator, of the above-specified kind are known from GB-A-2224213, the obturator in each case having flexibility to facilitate not only insertion of the obturator into the cannula prior to intubation, but also its easy and safe withdrawal once intubation is complete.

Many tracheostomy tubes used nowadays are of a form in which the cannula has two straight sections with an intermediate section that curves through, or substantially through, a right angle, and each obturator disclosed in GB-A-2224213 has flexible or hinge portions spaced from one another along its length to enable it to flex as it is inserted into and withdrawn from the tracheostomy tube. In this respect, the obturators involve a single-piece flat strip of a polymeric or other plastics material with spaced bead- or disc-like protuberances or segments projecting normally from it on both sides along part of its length. The strip readily flexes or hinges across its width within the spaces between the segments, so that it easily follows the curve of the cannula on insertion and withdrawal from the tracheostomy tube.

The forms of obturator disclosed in GB-A-2224213 can be used with especial advantage in the context of flexible tracheostomy tubes for paediatric use, but it is an object of the present invention to provide a combination of tracheostomy tube and obturator of the said above-specified kind that has advantage in a wider context, especially, for example, where the tracheostomy tube is of a less flexible form, for adult use.

According to the present invention a combination of tracheostomy tube and obturator of said above-specified kind is characterised in that the elongate member has a portion which along the length of that member from the distal end, is located beyond the flexible portion, and which projects beyond the shaft means in said plane to contact the inside of the wall of the cannula.

The projecting portion contacting the inside wall of the cannula has the advantage that it acts to resist collapse of the obturator within the cannula and tendency for its distal end to recede into the cannula during intubation. In both respects the risk of the cannula becoming occluded by the obturator during intubation is reduced, and resistance to the tendency for the distal end to recede assists the physician in correctly placing the tube in the patient's trachea.

The flexible portion of the obturator may be located between first and second sections of the shaft means which extend lengthwise of the elongate member from the proximal and distal ends respectively, and which are each dimensioned as aforesaid. The first and second sections of the shaft means may in these circumstances each comprise a flat part that has a cross-section corresponding to that of the flexible portion and is continuous with it, and a web-like part substantially normal to the flat part. The projecting portion for contacting the inside wall of the cannula may then be defined by a raised fin member on the web-like part.

An embodiment of a combination of tracheostomy tube and obturator in accordance with the present invention will now be described, by way of example, with reference to the accompanying drawings, in which:
Figure 1 is a perspective view of the obturator of the combination according to the invention;
Figures 2 and 3 are cross-sectional views taken on the lines II-II and III-III respectively of Figure 1; and
Figure 4 is a sectional side-elevation of the obturator of Figure 1 as inserted in a tracheostomy tube to form the combination according to the invention.

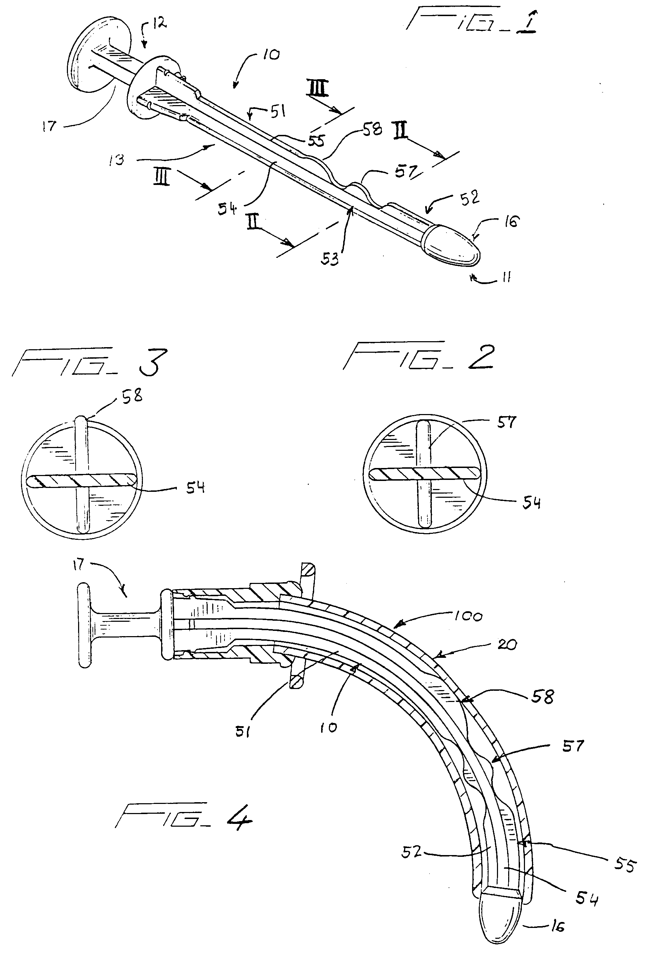

Referring to Figures 1 to 4, the obturator is a single-piece member 10 made of flexible material, preferably plastic, which includes a tip member 16 at the distal end 11 and grasping means 17, forming a handle at the proximal end 12. The member 10 includes shaft means 13 that extends between the distal and proximal ends 11 and 12 and is dimensioned to impart a degree of rigidity to it transversely throughout a substantial proportion of its length defined in first and second sections 51 and 52.

The shaft means 13 further includes a flexible section 53 which is positioned adjacent the distal end 11 between the sections 51 and 52. The flexible section 53, which flexes or hinges to follow the curvature of the tracheostomy tube 100, is of a substantially rectangular cross-section with a width as measured normal to the plane of curvature substantially greater than its thickness. The shaft means 13 is easily flexed in the plane of curvature at the first flexible portion 53, but is substantially stiff normal to that plane because of the greater width of its cross-section.

The rigid sections 51 and 52 each include a flat member 54 which has a rectangular cross-section corresponding to that of the flexible section 53 and is continuous with it. They also include a web-like member 55 which is substantially normal to the width of the first member 54.

The flexible section 53 includes a protuberance 57 that extends substantially normal to the width of the flexible section 53 and is spaced from the first and second rigid sections 51 and 52. Accordingly, the obturator flexes within the spaces between the protuberance 57 and the first and second sections 51 and 52. More protuberances may be included in the flexible section 53 spaced apart from one another and giving the obturator an extended length. Also, a different shape of protuberance from that of the protuberance 57 may be used; for example, circular-disc shapes or bead-like members similar to those used in the obturators described in GB-A-2224213, may be adopted.

The member 10 also has a portion 58 which along its length from the distal end 11 towards the proximal end 12 is located behind the flexible portion 53. The portion 58 is constituted by a raised fin member on the web-like member 55 of the section 51, and is dimensioned to project beyond the shaft means 13 in the plane of curvature such that when the obturator is inserted into the tracheostomy tube 100, the portion 58 contacts the inside of the wall of the cannula 20. This contact acts to resist collapse of the obturator within the cannula 20 and to hold the distal tip member 16 from receding into the cannula 20. This feature is extremely important since it prevents the tip member 16 from being pushed up inside the tracheostomy tube 100 while the physician is placing the tube in the patient.

The provision of the flexible section 53 within the obturator allows the physician to withdraw the obturator from the tube 100 without necessarily following the line of curvature of the cannula 20.

It will be understood that variations and modifications may be effected without departing from the scope of the novel concepts of the present invention.

## Claims

1. A combination of a tracheostomy tube (100) including a cannula and an obturator insertable into said cannula for guiding intubation of the tracheostomy tube (100), in which the cannula (20) of the tracheostomy tube (100) is curved with a non-constant radius and the obturator comprises an elongate, single-piece member (10) of resilient and flexible material that is bendable to follow the curve on its insertion in the cannula (20), the elongate member (10) involving shaft means (13) which extends between proximal and distal ends (11,12) of the member (10) and which over a substantial part of its length is dimensioned to impart a degree of rigidity both in the plane of the curve and normal to it, and in which the shaft means (13) includes a flexible portion (53) which is of substantially rectangular cross-section with a width measured normal to said plane greater than its thickness, and which is located adjacent the distal end (11) for easy flexing within said plane at that location, characterised in that the elongate member (10) has a portion (58) which along the length of that member (10) from the distal end (11), is located beyond the flexible portion (53), and which projects beyond the shaft means (13) in said plane to contact the inside of the wall of the cannula (20).

2. A combination according to Claim 1 wherein the flexible portion (53) is located between first and second sections (51,52) of the shaft means (13) which extend lengthwise of the elongate member (10) from the proximal and distal ends (11,12) respectively, and which are each dimensioned as aforesaid.

3. A combination according to claim 2 wherein the first and second sections (51,52) of the shaft means (13) each comprise a flat part (54) that has a cross-section corresponding to that of the flexible portion (53) and is continuous with it, and a web-like part (55) substantially normal to the flat part (54).

4. A combination according to Claim 3 wherein the portion that projects to contact the inside of the wall of the cannula (20) is defined by a raised fin member (58) on the web-like part (55) of said first section (51).

5. A combination according to any one of Claims 2 to 4 wherein the flexible portion (53) includes at least one protuberance (57) within said plane and having spaces between it and the first and second sections (51,52) so that the shaft means (13) flexes within the spaces.

## Patentansprüche

1. Kombination eines eine Kanüle umfassenden Trachealtubus (100) und eines in diese Kanüle einsetzbaren Obturators zur Führung beim Einsetzen des Trachealtubus (100), bei welcher die Kanüle (20) des Trachealtubus (100) mit einem nicht konstanten Radius gekrümmt ist und der Obturator ein längliches, einstückiges Glied (10) aus einem federnden und flexiblen Material umfaßt, welches biegbar ist, um bei seinem Einsetzen in die Kanüle (20) der Krümmung zu folgen, das längliche Glied (10) ein Schaftteil (13) umfaßt, welches sich zwischen den proximalen und distalen Enden (11, 12) des Glied (10) erstreckt und welches über einen wesentlichen Teil seiner Länge so dimensioniert ist, daß es sowohl in der Ebene der Krümmung als auch rechtwinklig dazu einen Grad von Steifigkeit aufweist und wobei das Schaftteil (13) einen flexiblen Teil (53) umfaßt, welcher im wesentlichen rechteckigen Querschnitt aufweist mit einer Breite, gemessen rechtwinklig zu dieser Ebene, die größer ist als seine Dicke, und welche nahe dem distalen Ende (11) zum leichten Verbiegen innerhalb dieser Ebene an dieser Stelle angeordnet ist, **dadurch gekennzeichnet**, daß das längliche Glied (10) einen Teil (58) aufweist, welcher längs der Länge dieses Glieds (10) vom distalen Ende (11) jenseits des flexiblen Teils (53) angeordnet ist und welches über das Schaftteil (13) in dieser Ebene übersteht, um die Innenseite der Wandung der Kanüle (20) zu berühren.

2. Kombination nach Anspruch 1, **wobei** der flexible Teil (53) zwischen ersten und zweiten Abschnitten (51, 52) des Schaftteils (13) angeordnet ist, welches sich längs des länglichen Glieds (10) von den proximalen bzw. distalen Enden (11, 12) erstrecken und welche jeweils wie vorerwähnt dimensioniert sind.

3. Kombination nach Anspruch 2, **wobei** die erste und zweiten Abschnitte (51, 52) des Schaftteils (13) jeweils aus einem flachen Teil (54), das einen Querschnitt entsprechend demjenigen des flexiblen Teils (53) aufweisen und welches verlängert mit diesem ist, und aus einem blattartigen Teil (55) im wesentlichen rechtwinklig zum flachen Teil (54) bestehen.

4. Kombination nach Anspruch 3, **wobei** das Teil, welches zur Berührung mit der Innenseite der Wand der Kanüle (20) übersteht, gebildet wird durch ein erhabenes Flossenteil (58) des blattartigen Teils (55) des ersten Abschnitts (51).

5. Kombination nach einem der Ansprüche 2 bis 4, **wobei** der flexible Teil (53) mindestens einen Höcker (57) innerhalb dieser Ebene umfaßt und welches Zwischenräume zwischen sich und den ersten und zweiten Abschnitten (51, 52) aufweist, so daß das Schaftteil (13) innerhalb dieser Zwischenräume biegbar ist.

## Revendications

1. Combinaison d'un tube de trachéotomie (100) comportant une canule et d'un obturateur pouvant être inséré dans ladite canule pour guider l'intubation du tube de trachéotomie (100), dans laquelle la canule (20) du tube de trachéotomie (100) est incurvée en ayant un rayon non-constant et l'obturateur comporte un élément allongé en une seule pièce (10) constitué d'un matériau élastique et souple qui peut être courbé pour suivre la courbure lors de son insertion dans la canule (20), l'élément allongé (10) entraînant des moyens formant arbre (13) qui s'étendent entre les extrémités proximale et distale (11, 12) de l'élément (10) et qui, sur une partie importante de leur longueur, sont dimensionnés pour entraîner un degré de rigidité dans le plan de la courbe et dans le plan perpendiculaire à celui-ci, et dans laquelle les moyens formant arbre (13) comportent une partie souple (53) qui a une section transversale pratiquement rectangulaire ayant une largeur, mesurée perpendiculairement audit plan, plus grande que son épaisseur, et qui est située adjacente à l'extrémité distale (11) pour s'infléchir facilement dans ledit plan à cet emplacement, caractérisée en ce que l'élément allongé (10) a une partie (58) qui, sur la longueur de cet élément (10) à partir de l'extrémité distale (11), est située au-delà de la partie souple (53), et qui fait saillie au-delà des moyens formant arbre (13) dans ledit plan pour venir en contact avec l'intérieur de la paroi de la canule (20).

2. Combinaison selon la revendication 1, dans laquelle la partie souple (53) est située entre des premier et second tronçons (51, 52) des moyens formant arbre (13) qui s'étendent dans le sens de la longueur de l'élément allongé (10) à partir des extrémités proximale et distale (11, 12) respectivement, et qui sont chacun dimensionnés comme mentionné ci-dessus.

3. Combinaison selon la revendication 2, dans laquelle les premier et second tronçons (51, 52) des moyens formant arbre (13) comportent chacun une partie plate (54) qui a une section transversale correspondant à celle de la partie souple (53) et qui est continue avec celle-ci, et une partie analogue à une bande (55) pratiquement perpendiculaire à la partie plate (54).

4. Combinaison selon la revendication 3, dans laquelle la partie qui fait saillie pour venir en contact avec la partie intérieure de la paroi de la canule (20) est définie par un élément formant ailette surélevée (58) situé sur la partie analogue à une bande (55) dudit premier tronçon (51).

5. Combinaison selon l'une quelconque des revendications 2 à 4, dans laquelle la partie souple (53) comporte au moins une saillie (57) située dans ledit plan et ayant des espaces entre celle-ci et les premier et second tronçons (51, 52) de sorte que les moyens formant arbre (13) s'infléchissent dans les espaces.
